# EUROPÄISCHE PATENTSCHRIFT

(11) **EP 4 358 883 B1**
(45) Veröffentlichungstag und Bekanntmachung des Hinweises auf die Patenterteilung: **14.05.2025**
(21) Anmeldenummer: 22737439.4
(22) Anmeldetag: 22.06.2022
(51) Int. Cl.: A61B 18/14, A61B 18/00

(54) **BETÄTIGUNGSELEMENT, CHIRURGISCHES INSTRUMENT UND VERFAHREN ZUR HERSTELLUNG DES BETÄTIGUNGSINSTRUMENTS**
ACTUATING ELEMENT, SURGICAL INSTRUMENT, AND METHOD FOR MANUFACTURING THE ACTUATING INSTRUMENT
ÉLÉMENT D'ACTIONNEMENT, INSTRUMENT CHIRURGICAL ET PROCÉDÉ DE FABRICATION DE L'INSTRUMENT D'ACTIONNEMENT

(30) Priorität: 23.06.2021 DE 102021116216
(43) Veröffentlichungstag der Anmeldung: 01.05.2024
(73) Patentinhaber: Karl Storz SE & Co. KG, 78532 Tuttlingen (DE)
(72) Erfinder: PÖNISCH, Judith, 78532 Tuttlingen (DE); KÄRCHER, Daniel, 78532 Tuttlingen (DE); LÄNGLE, Dominik, 78532 Tuttlingen (DE); MERZ, Robin, 78532 Tuttlingen (DE); SCHNEIDER, Janosz, 78532 Tuttlingen (DE); SCHNEIDER, Sven, 78532 Tuttlingen (DE); UNGER, Tobias, 78532 Tuttlingen (DE)
(86) Internationale Anmeldenummer: PCT/EP2022/066938
(87) Internationale Veröffentlichungsnummer: WO 2022/268846

(56) Entgegenhaltungen:
- CN-U- 209 404 943
- DE-A1- 102014 116 065
- DE-A1- 4 222 769
- US-A1- 2012 116 262

## Beschreibung

### GEBIET DER ERFINDUNG

Die vorliegende Erfindung betrifft ein Kraftübertragungselement mit kombinierter mechanischer Lagerung und elektrischer Trennung, sowie ein chirurgisches Instrument umfassend das Kraftübertragungselement und ein Verfahren zur Herstellung des Kraftübertragungselements.

### TECHNISCHER HINTERGRUND

Chirurgische Instrumente weisen oftmals eine mechanische Lagerung, insbesondere eine Verdrehsicherung oder eine Axialführung mit Anschlag, auf. Zudem weisen chirurgische Instrumente oftmals eine elektrische Trennung (Isolierung) auf. Die mechanische Lagerung und elektrische Trennung werden durch separate Bauteile oder Elemente des chirurgischen Instruments realisiert.

Beispielsweise weisen chirurgische Rohrschaftinstrumente (z. B. endoskopische Instrumente) mit einem Rohrschaft und einem darin gelagerten Kraftübertragungselement eine Verdrehsicherung auf, die ein Verdrehen des Kraftübertragungselements gegenüber dem Rohrschaft verhindert. Zudem können derartige chirurgische Rohrschaftinstrumente zusätzlich oder alternativ eine Axialführung mit Anschlag aufweisen, die das Kraftübertragungselement in dem Rohrschaft, bevorzugt zentral, in axialer Richtung führt und dessen translatorische Bewegung mittels eines oder zweier Anschläge begrenzt. DE 10 2014 116065 A1 offenbart ein bipolares chirurgisches Instrument mit einem mehrfach verwendbaren Handgriff und einem einmal verwendbaren Werkzeug sowie einer Verbindungsvorrichtung dazwischen.

Weiter beispielsweise weisen bipolar betriebene chirurgische Rohrschaftinstrumente (z. B. endoskopische Instrumente mit bipolarem Werkzeug / Zubehör) zwei elektrische Leitungen auf, die üblicherweise durch zwei gegeneinander elektrisch isolierte Bauteile oder Elemente des chirurgischen Rohrschaftinstruments gebildet sind. Bei bipolar betriebenen chirurgischen Rohrschaftinstrumenten sind an dem Zubehör des chirurgischen Rohrschaftinstruments zwei Elektroden (Aktivelektrode und Neutralelektrode) angeordnet. Dabei kann von einer ersten Elektrode (Aktivelektrode) aus hochfrequenter Wechselstrom direkt gegenüber der zweiten Elektrode (Neutralelektrode) in das Zielgewebe eingebracht werden.

Bei bipolar betriebenen chirurgischen Rohrschaftinstrumenten mit einem Rohrschaft, der als eine erste elektrische Leitung ausgebildet ist, und einem darin gelagerten Kraftübertragungselement, das als eine zweite elektrische Leitung ausgebildet ist, ist zum einen eine Verdrehsicherung oder Axialführung für das Kraftübertragungselement in dem Rohrschaft vorgesehen und zum anderen eine separate elektrische Trennung bzw. Isolierung des Kraftübertragungselements gegenüber Rohrschaft vorgesehen. Somit sind zusätzliche Einzelteile und Verbindungen in derartigen chirurgischen Rohrschaftinstrumenten angeordnet, um beide Funktionen, die mechanische Führung und die elektrische Trennung, zu realisieren.

### ZUSAMMENFASSUNG DER ERFINDUNG

Vor diesem Hintergrund liegt der vorliegenden Erfindung die Aufgabe zugrunde, ein verbessertes medizinisches Instrument mit kombinierter mechanischer Lagerung und elektrischer Trennung bereitzustellen.

Erfindungsgemäß wird diese Aufgabe durch ein Betätigungselement mit den Merkmalen des Patentanspruchs 1 und/oder durch ein chirurgisches Instrument mit den Merkmalen des Patentanspruchs 9 und/oder durch ein Verfahren mit den Merkmalen des Patentanspruchs 12 gelöst.

Dementsprechend ist gemäß einem ersten Aspekt der vorliegenden Erfindung ein Kraftübertragungselement für chirurgische Instrumente mit einem Stab, einer elektrischen Isolierung und einer Hülse vorgesehen. Der Stab ist elektrisch leitfähig und zur Kraftübertragung ausgebildet. Die elektrische Isolierung erstreckt sich umfänglich über eine äußere Oberfläche und zumindest abschnittsweise in axialer Richtung entlang des Stabs. Die Hülse ist elektrisch leitfähig. Die Hülse ist umfänglich um die elektrische Isolierung angeordnet und erstreckt sich zumindest abschnittsweise in axialer Richtung entlang der elektrischen Isolierung. Der Stab weist zumindest eine Vertiefung auf. Die Isolierung erstreckt sich zumindest entlang der Vertiefung. Die Hülse weist eine Prägung auf, die sich zumindest abschnittsweise in die Vertiefung erstreckt und zum Eingriff mit einem Sicherungselement eines chirurgischen Instruments ausgebildet ist.

Ferner ist gemäß einem zweiten Aspekt der vorliegenden Erfindung ein chirurgisches Instrument, insbesondere ein chirurgisches Rohrschaftinstrument mit einem Kraftübertragungselement gemäß dem ersten Aspekt der vorliegenden Erfindung, einem Rohrschaft, einem Sicherungselement, einem Werkzeug und einer Betätigungsschnittstelle vorgesehen. In dem Rohrschaft ist das Kraftübertragungselement aufgenommen. Das Sicherungselement ist in dem Rohrschaft gelagert und greift in die Prägung der Hülse des Kraftübertragungselements ein. Das Werkzeug ist mittels des Kraftübertragungselements bewegbar. Die Betätigungsschnittstelle ist zum Betätigen des Kraftübertragungselements ausgebildet.

Außerdem ist gemäß einem dritten Aspekt der vorliegenden Erfindung ein Verfahren zur Herstellung eines Kraftübertragungselements für chirurgische Instrumente, insbesondere eines Kraftübertragungselements gemäß dem ersten Aspekt der vorliegenden Erfindung, vorgesehen, das die folgenden Schritte umfasst:
Bereitstellen eines elektrisch leitfähigen zur Kraftübertragung ausgebildeten Stabs, der eine Vertiefung aufweist.
Aufbringen einer elektrischen Isolierung umfänglich über eine äußere Oberfläche und zumindest abschnittsweise in axialer Richtung entlang des Stabs, sodass die Isolierung sich zumindest entlang der Vertiefung erstreckt.
Umfängliches Anordnen einer elektrisch leitfähigen Hülse um die Isolierung zumindest abschnittsweise in axialer Richtung entlang der elektrischen Isolierung.
Einprägen einer zumindest abschnittsweise in die Vertiefung eingreifenden und zum Eingriff mit einem Sicherungselement eines chirurgischen Instruments ausgebildeten Prägung in die Hülse.

Der Stab kann zumindest teilweise aus einem metallischen Werkstoff wie beispielsweise Stahl oder Edelstahl gefertigt sein. Der Stab kann insbesondere ausgebildet sein, eine Kraft in axialer Richtung (Zug oder Druck) und zusätzlich oder alternativ ein Drehmoment zu Übertragen. Die Kraft in axialer Richtung bzw. Das Drehmoment kann von der Betätigungsschnittstelle auf den Stab übertragen werden und durch den Stab an ein Zubehör oder Werkzeug des chirurgischen Instruments weitergeleitet werden, um das Zubehör bzw. Werkzeug zu bewegen.

Die Vertiefung in dem Stab dient der Beschränkung oder Sperrung wenigstens eines translatorischen oder rotatorischen Freiheitsgrads des Stabs gegenüber der Hülse. Dabei wird der Stab gegenüber der Hülse im Bereich der Vertiefung und der dort eingreifenden Prägung gelagert und zusätzlich oder alternativ geführt. Die Vertiefung kann durch ein spanendes Verfahren wie beispielsweise Fräsen in den Stab eingebracht werden.

Die Isolierung kann zumindest teilweise aus einem polymeren Werkstoff wie beispielsweise Polytetrafluorethylen (PTFE, Teflon) oder einem keramischen Werkstoff gefertigt sein. Die Isolierung trennt den elektrisch leitenden Stab elektrisch von der elektrisch leitenden Hülse. Insbesondere in einem bipolaren Betrieb des chirurgischen Werkzeugs kann der elektrische Strom über das Kraftübertragungselement geleitet werden, indem einer der beiden Pole zur Leitung des elektrischen Stroms mit dem Stab und der andere der beiden Pole zur Leitung des elektrischen Stroms mit der durch die elektrische Isolierung von dem Stab elektrisch getrennten Hülse elektrisch verbunden wird.

Die Isolierung ist umfänglich auf der Oberfläche des Stabs angeordnet. Dabei ist die Isolierung zumindest in einem Abschnitt entlang des Stabs angeordnet, der sich zumindest über einen Teil der Vertiefung erstreckt. Die Isolierung berührt die Oberfläche des Stabs und auch im Bereich der Vertiefung ist die Isolierung in Kontakt mit der Oberfläche der Vertiefung bzw. des Stabs. Insbesondere ist die Isolierung fest mechanisch (z. B. Presspassung) mit dem Stab verbunden. Die Isolierung wird auf dem Stab somit derart aufgebracht, dass sie zumindest im Bereich der Vertiefung den Stab umfänglich umschließt. Das Aufbringen kann beispielsweise durch ein Beschichten oder ein Aufschrumpfen eines Schrumpfschlauchs erfolgen. Die Isolierung kann entlang des Umfangs des Stabs eine im Wesentlichen konstante Dicke aufweisen. Dementsprechend greift die Prägung der Hülse derart in die Vertiefung des Stabs ein, dass die Prägung mit der Isolierung jedoch nicht mit dem Stab in Kontakt steht.

Die Hülse kann zumindest teilweise aus einem metallischen Werkstoff wie beispielsweise Stahl oder Edelstahl gefertigt sein. Die Hülse ist umfänglich in einem Bereich um den Stab mit Isolierung angeordnet, der zumindest einen Abschnitt der Vertiefung umfasst. Die Bohrung der Hülse in axialer Richtung ist insbesondere konzentrisch angeordnet. Die Hülse ist mit der Isolierung entweder fest mechanisch verbunden (z. B. Presspassung) oder ermöglicht eine Verschiebung bzw. Rotation des Stabs mit der Isolierung gegenüber der Hülse mit einer vordefinierten Gleitreibung (z. B. Spielpassung) in der Bohrung der Hülse. Dazu wird die Hülse auf den Stab aufgeschoben und soweit vorgeschoben, dass sie zumindest teilweise in dem Bereich der Vertiefung angeordnet ist.

Die Prägung wird derart in die Hülse eingeprägt, dass die Prägung in die Vertiefung eingreift, wobei die Prägung nur die Isolierung um den Stab und nicht den Stab selbst kontaktiert. Insbesondere gereift die Prägung in radialer Richtung in die Vertiefung ein. Die Prägung und die Vertiefung bilden dabei ein Lager oder eine Führung für den Stab gegenüber der Hülse, wobei die Isolierung mit beiden in Kontakt steht und beide voneinander trennt. Der Stab ist daher mit der Isolierung in der Hülse zumindest im Bereich der Vertiefung und der darin eingreifenden Prägung gelagert bzw. geführt. Das Einprägen der Hülse kann beispielsweise durch einen Stempel in einem vordefinierten Abschnitt des Umfangs der Hülse oder vollumfänglich durch ein Rollprägen erfolgen.

Das Kraftübertragungselement ist in den Rohrschaft des chirurgischen Instruments eingeschoben. Dabei ist das Kraftübertragungselement zumindest über die Hülse in dem Rohrschaft gelagert bzw. geführt. Die Hülse kann gegenüber dem Rohrschaft elektrisch isoliert sein. Insbesondere ist die Hülse in dem Rohrschaft derart gelagert bzw. geführt, dass das sie bzw. das Kraftübertragungselement sich gegenüber dem Rohrschaft axial verschieben oder rotieren kann. Somit kann das Kraftübertragungselement eine axiale Kraft (Zug oder Druck) und zusätzlich oder alternativ ein Drehmoment innerhalb des Rohrschafts und relativ zu diesem übertragen.

Um ein ungewolltes Verschieben oder Verdrehen des Kraftübertragungselements gegenüber dem Rohrschaft, das beispielsweise zu einem Herausrutschen des Kraftübertragungselements aus dem Rohrschaft führen kann, zu verhindern, ist die Hülse und somit das Kraftübertragungselement in dem Rohrschaft über das Sicherungselement gesichert. Dazu greift das in dem Rohrschaft gelagerte Sicherungselement in die Prägung der Hülse ein. Beispielsweise kann das Sicherungselement in radialer Richtung gegenüber dem Rohrschaft beweglich gelagert sein und in radialer Richtung in die Prägung eingreifen. Insbesondere kann das Sicherungselement in radialer Richtung vorgespannt sein, beispielsweise mittels eines elastischen Federrings.

Das Werkzeug, das insbesondere in Form eines austauschbaren Zubehörs ausgebildet sein kann, wird durch die über das Kraftübertragungselement innerhalb und gegenüber des Rohrschafts übertragene axiale Kraft bzw. Drehmoment bewegt. Das Werkzeug kann als Klemme, Zange, Pinzette, Greifer, Schere und dergleichen ausgebildet sein. Insbesondere kann das Werkzeug zudem ausgebildet sein, bipolar betrieben zu werden, um beispielsweise Gewebe zu schneiden, zu veröden und dergleichen.

Die Betätigungsschnittstelle dient zum Betätigen des Kraftübertragungselements, indem eine axiale Kraft oder ein Drehmoment auf das Kraftübertragungselement aufgebracht wird.

Sofern das chirurgische Instrument durch einen Nutzer wie beispielsweise einen Chirurgen geführt werden soll, umfasst das chirurgische Instrument einen Handgriff als Handhabe für den Nutzer. Die Betätigungsschnittstelle überträgt sodann eine Kraft bzw. ein Drehmoment, die/das von dem Nutzer am Handgriff über eine geeignete Betätigung (z. B. beweglicher Griffschenkel) aufgebracht wird, an das Kraftübertragungselement.

Sofern das chirurgische Instrument an einen Roboter anschließbar ist, umfasst das chirurgische Instrument eine entsprechende Verbindungsschnittstelle für den Roboter, der eine axiale Kraft bzw. ein Drehmoment über die Betätigungsschnittstelle auf das Kraftübertragungselement aufbringen kann.

Das erfindungsgemäße Kraftübertragungselement mit Stab, Isolierung und Hülse lässt sich besonders einfach herstellen, da lediglich die Isolierung auf dem Stab aufgebracht, die Hülse sodann darauf angeordnet und schließlich die Prägung im Bereich der Vertiefung des Stabs eingeprägt werden muss. Zudem kann durch das erfindungsgemäße Kraftübertragungselement auf ein separates Bauteil zum Führen und elektrischen Isolieren des Kraftübertragungselements gegenüber dem Rohrschaft des chirurgischen Instruments verzichtet werden. Vorteilhaft werden somit erhöhte Produktions- und Fertigungskosten aufgrund zusätzlicher Einzelteile und Verbindungen vermieden. Ferner wird auch ein Ausfall des betroffenen chirurgischen Rohrschaftinstruments durch mechanische Belastung besagter Verbindungen, die üblicherweise stark mechanisch beansprucht werden, vorteilhaft vermieden.

Vorteilhafte Weiterbildungen und Ausgestaltungen der vorliegenden Erfindung sind Gegenstand der entsprechenden abhängigen Patentansprüche.

Gemäß einer Weiterbildung der vorliegenden Erfindung ist der Stab des Kraftübertragungselements als Rundstab ausgebildet. Die elektrische Isolierung erstreckt sich zumindest abschnittsweise zirkulär entlang der Oberfläche des Stabs. Die Hülse ist als Rundrohr ausgebildet und erstreckt sich zumindest abschnittsweise zirkulär entlang der Oberfläche der elektrischen Isolierung.

Gemäß einer weiteren Weiterbildung ist der Rohrschaft als Rundrohr ausgebildet. Der Stab des Kraftübertragungselements ist als Rundstab ausgebildet. Die elektrische Isolierung erstreckt sich zumindest abschnittsweise zirkulär entlang der Oberfläche des Stabs. Die Hülse ist als Rundrohr ausgebildet und sich erstreckt zumindest abschnittsweise zirkulär entlang der Oberfläche der elektrischen Isolierung. Das Kraftübertragungselement ist konzentrisch in dem Rohrschaft angeordnet.

Der als Rundstab ausgebildete Stab hat einen im Wesentlichen kreisrunden Querschnitt mit im Wesentlichen konstanten Durchmesser entlang der axialen Richtung, außer im Bereich der Vertiefung.

Die Isolierung ist, insbesondere abseits der Vertiefung, zirkulär und insbesondere konzentrisch um den Stab angeordnet.

Die als Rundrohr ausgebildete Hülse hat einen im Wesentlichen kreisrunden Querschnitt mit im Wesentlichen konstanten Durchmesser entlang der axialen Richtung, außer im Bereich der Prägung. Die als Rundrohr ausgebildete Hülse ist zirkulär und insbesondere konzentrisch um die Isolierung angeordnet.

Der als Rundrohr ausgebildete Rohrschaft hat einen im Wesentlichen kreisrunden Querschnitt mit im Wesentlichen konstanten Durchmesser entlang der axialen Richtung. Das Kraftübertragungselement mit als Rundstab ausgebildetem Stab und als Rundrohr ausgebildeter Hülse ist in dem als Rundrohr ausgebildeten Rohrschaft konzentrisch angeordnet. Dabei greift das Sicherungselement in radialer Richtung in die Prägung ein, um das Kraftübertragungselement mit dem als Rundstab ausgebildeten Stab und der als Rundrohr ausgebildeten Hülse in dem als Rundrohr ausgebildeten Rohrschaft zu sichern.

Das derart ausgebildete Kraftübertragungselement und chirurgische Instrument sind vorteilhaft besonders einfach zu fertigen.

Gemäß einer Weiterbildung ist die Prägung zum Sperren wenigstens des rotatorischen Freiheitsgrads um eine Achse parallel zu der Hülse bzw. der axialen Richtung der Hülse bei Eingriff eines Sicherungselements ausgebildet.

Das Sicherungselement des chirurgischen Elements greift derart in die Prägung ein, dass eine Rotation der Hülse und je nach Ausgestaltung der Vertiefung auch des Stabs gegenüber dem Rohrschaft blockiert ist. Somit ist höchstens eine axiale Verschiebung der Hülse und je nach Ausgestaltung der Vertiefung auch des Stabs gegenüber dem Rohrschaft möglich.

Somit können ungewollte Rotationen der Hülse bzw. des gesamten Kraftübertragungselements auch gegenüber dem Werkzeug bzw. Zubehör des chirurgischen Elements vermieden und somit eine einwandfreie Funktion des Werkzeugs/Zubehörs sichergestellt werden.

Gemäß einer Weiterbildung weist die Prägung wenigstens einen im Wesentlichen ebenen Abschnitt zur im Wesentlichen plan anliegenden Kontaktierung einer ebenen Fläche eines Sicherungselements auf. Der im Wesentlichen ebene Abschnitt verläuft parallel zu der axialen Richtung der Hülse und liegt gegenüber der äußeren Oberfläche der Hülse radial weiter innen.

Der im Wesentlichen ebene Abschnitt kann in dem Stab bzw. Rundstab beispielsweise durch Planfräsen geschaffen werden. Der im Wesentlichen ebene Abschnitt bildet eine ebene Kontaktfläche. Wird dieser von der ebenfalls im Wesentlichen ebenen Fläche des Sicherungselements, das in radialer Richtung nach innen auf die Hülse vorgespannt ist, kontaktiert, vorzugsweise plan kontaktiert, so ist die Hülse und je nach Ausgestaltung der Vertiefung auch der Stab gegenüber dem Rohrschaft des chirurgischen Instruments gegen Rotation/Verdrehen gesichert.

Diese Ausgestaltung der Prägung ermöglicht somit vorteilhaft eine besonders einfach zu fertigende Verdrehsicherung.

Gemäß einer Weiterbildung der vorliegenden Erfindung weist die Vertiefung wenigstens einen im Wesentlichen ebenen Abschnitt parallel zu der axialen Richtung des Stabs auf. Der im Wesentlichen ebene Abschnitt liegt gegenüber der äußeren Oberfläche des Stabs in radialer Richtung weiter innen und ist mit dem im Wesentlichen ebenen Abschnitt der Prägung aneinander anliegend, insbesondere plan anliegend, kontaktiert.

Sofern die Hülse über den im Wesentlichen ebenen Abschnitt der Prägung und das Sicherungselement mit im wesentlichen ebener Fläche des chirurgischen Elements gegen Rotation bzw. Verdrehen im Rohrschaft gesichert ist, so ist auch der Stab über seinen im Wesentlichen ebenen Abschnitt und über den im Wesentlichen ebenen Abschnitt der Prägung gegenüber dem Rohrschaft gegen Rotation/Verdrehen gesichert.

Gemäß einer Weiterbildung der vorliegenden Erfindung ist die Prägung zum Begrenzen oder Sperren wenigstens des translatorischen Freiheitsgrads in einer Richtung parallel zu der Hülse bzw. der axialen Richtung der Hülse bei Eingriff eines Sicherungselements ausgebildet.

Das Sicherungselement des chirurgischen Elements greift derart in die Prägung ein, dass eine axiale Verschiebung der Hülse und je nach Ausgestaltung der Vertiefung auch des Stabs gegenüber dem Rohrschaft begrenzt oder blockiert ist. Bei einer Ausführungsform kann eine Rotation der Hülse und je nach Ausgestaltung der Vertiefung auch des Stabs gegenüber dem Rohrschaft ermöglicht sein.

Somit können ungewollte axiale Verschiebungen der Hülse bzw. des gesamten Kraftübertragungselements auch gegenüber dem Werkzeug bzw. Zubehör des chirurgischen Elements vermieden und somit eine einwandfreie Funktion des Werkzeugs/Zubehörs sichergestellt werden.

Gemäß einer Weiterbildung weist die Prägung wenigstens einen Anschlag auf. Der wenigstens eine Anschlag der Prägung ist zum Sperren oder Begrenzen wenigstens des translatorischen Freiheitsgrads parallel zu der Hülse bzw. der axialen Richtung der Hülse bei Eingriff des Sicherungselements ausgebildet.

Wenn das Sicherungselement mit wenigstens einer seiner Flanken in axialer Richtung an dem Anschlag der Prägung anliegt, ist keine axiale Verschiebung der Hülse gegenüber dem Rohrschaft (in Richtung mit dem Anschlag auf das Sicherungselement zu) mehr möglich. Insbesondere wenn zwei sich in axialer Richtung gegenüber liegende Anschläge der Prägung vorgesehen sind, ist die axiale Bewegung der Hülse gegenüber dem Rohrschaft in beide axialen Bewegungsrichtungen zumindest begrenzt. Sind die beiden sich in axialer Richtung gegenüberliegenden Anschläge derart angeordnet, dass sie immer an den beiden in axialer Richtung ausgerichteten Flanken des Sicherungselements anliegen, so ist der translatorische Freiheitsgrad der Hülse gegenüber dem Rohrschaft blockiert und nicht nur begrenzt.

Gemäß einer Weiterentwicklung der vorliegenden Erfindung weist die Vertiefung wenigstens einen Anschlag auf. Der wenigstens eine Anschlag der Vertiefung ist zum Begrenzen oder Sperren wenigstens des translatorischen Freiheitsgrads in einer Richtung parallel zu dem Stab bzw. zur axialen Richtung des Stabs gemeinsam mit der Prägung ausgebildet.

Wenn die Prägung mit ihrem wenigstens einen Anschlag in axialer Richtung an dem Anschlag der Vertiefung anliegt, ist keine axiale Verschiebung des Stabs gegenüber der Hülse in Richtung mit dem Anschlag der Vertiefung auf den Anschlag der Prägung zu mehr möglich. Insbesondere wenn zwei sich in axialer Richtung gegenüber liegende Anschläge der Vertiefung vorliegen, ist die axiale Bewegung des Stabs gegenüber der Hülse in beide axialen Bewegungsrichtungen zumindest begrenzt. Sind die beiden sich in axialer Richtung gegenüberliegenden Anschläge der Vertiefung derart angeordnet, dass sie gleichermaßen an den beiden Anschlägen der Prägung anliegen, so ist der translatorische Freiheitsgrad des Stabs gegenüber der Hülse blockiert und nicht nur begrenzt.

Gemäß einer Weiterbildung der vorliegenden Erfindung weist die elektrische Isolierung zumindest im Bereich der Prägung Gleiteigenschaften auf ihrer äußeren Oberfläche auf. Zusätzlich oder alternativ weist die Hülse zumindest im Bereich der Prägung Gleiteigenschaften auf ihrer inneren Oberfläche auf.

Die Gleiteigenschaften der äußeren Oberfläche der elektrischen Isolierung bzw. der inneren Oberfläche der Prägung können durch geeignete Wahl des Werkstoffs (z. B. PTFE für die elektrische Isolierung) und zusätzlich oder alternativ durch geeignete Behandlung der jeweiligen Oberfläche (z. B. Polieren, Honen usw.) sichergestellt werden. Beispielsweise kann die elektrische Isolierung aus PTFE und die Hülse aus Edelstahl gefertigt sein. Zudem kann die innere Oberfläche der Hülse poliert sein. Die polierte innere Oberfläche der Hülse aus Edelstahl kann sodann auf der äußeren Oberfläche der Isolierung aus PTFE gut gleiten, sodass eine gleitende axiale Verschiebung oder Rotation der Hülse gegenüber der elektrischen Isolierung möglich ist.

Gemäß einer Weiterbildung der vorliegenden Erfindung weist das Sicherungselement auf seiner der Prägung zugewandten Oberfläche Gleiteigenschaften auf. Zusätzlich oder alternativ weist die Prägung auf ihrer äußeren Oberfläche Gleiteigenschaften auf.

Die Gleiteigenschaften der der Prägung zugewandten Oberfläche des Sicherungselements bzw. der äußeren Oberfläche der Prägung können durch geeignete Wahl des Werkstoffs und zusätzlich oder alternativ durch geeignete Behandlung der jeweiligen Oberfläche (z. B. Polieren, Honen usw.) sichergestellt werden. Beispielsweise können die Hülse und das Sicherungselement aus Edelstahl gefertigt und zudem die äußere Oberfläche der Hülse und die der Prägung zugewandte Oberfläche des Sicherungselements poliert sein. Die polierte äußere Oberfläche der Hülse aus Edelstahl kann sodann auf der der Prägung zugewandten Oberfläche des Sicherungselements aus Edelstahl gut gleiten, sodass eine gleitende axiale Verschiebung oder Rotation der Hülse gegenüber dem Sicherungselement bzw. dem Rohrschaft des chirurgischen Instruments möglich ist.

Gemäß einer Weiterbildung der vorliegenden Erfindung der Schritt des Bereitstellens einen Schritt des Schaffens der Vertiefung in dem Stab, insbesondere durch Umformen, beispielweise Pressen, oder durch Zerspanen, beispielsweise Fräsen oder Drehen.

Gemäß einer Weiterbildung der vorliegenden Erfindung umfasst der Schritt des Aufbringens der Isolierung ein Aufschrumpfen eines Schrumpfschlauchs auf den Stab oder ein Beschichten des Stabs.

Gemäß einer Weiterbildung der vorliegenden Erfindung umfasst der Schritt des umfänglichen Anordnens die folgenden Schritte:
Erwärmen der Hülse bis durch thermische Dehnung ein Innendurchmesser der Hülse größer als ein Außendurchmesser der elektrischen Isolierung ist.
Aufschieben der erwärmten Hülse auf die elektrische Isolierung.
Abkühlen der erwärmten und aufgeschobenen Hülse.

Die erwärmte Hülse wird soweit auf den Stab mit der elektrischen Isolierung aufgeschoben, dass sie zumindest teilweise in einem Bereich der Vertiefung des Stabs angeordnet ist. Sobald die aufgeschobene Hülse abgekühlt ist, besteht zwischen der Hülse und der elektrischen Isolierung eine Presspassung, also feste mechanische Verbindung, oder eine Spielpassung, also eine Gleitverbindung.

### INHALTSANGABE DER ZEICHNUNG

Die vorliegende Erfindung wird nachfolgend anhand der in den schematischen Figuren der Zeichnung angegebenen Ausführungsbeispiele näher erläutert. Es zeigen dabei:
- Fig. 1: eine Seitenansicht eines handgeführten chirurgischen Instruments;
- Fig. 2: einen Längsschnitt durch das chirurgische Instrument;
- Fig. 3: eine Isometrische Ansicht einer ersten Ausführungsform des Kraftübertragungselements des chirurgischen Instruments;
- Fig. 4A: einen Längsschnitt durch eine erste Variante der ersten Ausführungsform des Kraftübertragungselements;
- Fig. 4B: einen Längsschnitt durch eine zweite Variante der ersten Ausführungsform des Kraftübertragungselements;
- Fig. 4C: einen Querschnitt durch die erste Ausführungsform des Kraftübertragungselements im Bereich der Prägung;
- Fig. 5: eine Isometrische Ansicht einer zweiten Ausführungsform des Kraftübertragungselements;
- Fig. 6A: einen Längsschnitt durch eine erste Variante der zweiten Ausführungsform des Kraftübertragungselements;
- Fig. 6B: einen Längsschnitt durch eine zweite Variante der zweiten Ausführungsform des Kraftübertragungselements;
- Fig. 6C: einen Querschnitt durch die zweite Ausführungsform des Kraftübertragungselements im Bereich der Prägung;
- Fig. 7: eine Isometrische Ansicht einer dritten Ausführungsform des Kraftübertragungselements;
- Fig. 8A: einen Längsschnitt durch eine erste Variante der dritten Ausführungsform des Kraftübertragungselements;
- Fig. 8B: einen Längsschnitt durch eine zweite Variante der dritten Ausführungsform des Kraftübertragungselements;
- Fig. 8C: einen Querschnitt durch die dritte Ausführungsform des Kraftübertragungselements im Bereich der Prägung;
- Fig. 9: eine Isometrische Ansicht einer vierten Ausführungsform des Kraftübertragungselements;
- Fig. 10: einen Längsschnitt durch die vierte Ausführungsform des Kraftübertragungselements; und
- Fig. 11: ein Ablaufdiagramm einer Ausführungsform des Verfahrens zur Herstellung eines Kraftübertragungselements.

Die beiliegenden Figuren der Zeichnung sollen ein weiteres Verständnis der Ausführungsformen der Erfindung vermitteln. Sie veranschaulichen Ausführungsformen und dienen im Zusammenhang mit der Beschreibung der Erklärung von Prinzipien und Konzepten der Erfindung. Andere Ausführungsformen und viele der genannten Vorteile ergeben sich im Hinblick auf die Zeichnungen. Die Elemente der Zeichnungen sind nicht notwendigerweise maßstabsgetreu zueinander gezeigt.

In den Figuren der Zeichnung sind gleiche, funktionsgleiche und gleich wirkende Elemente, Merkmale und Komponenten - sofern nichts Anderes ausgeführt ist - jeweils mit denselben Bezugszeichen versehen.

### BESCHREIBUNG VON AUSFÜHRUNGSBEISPIELEN

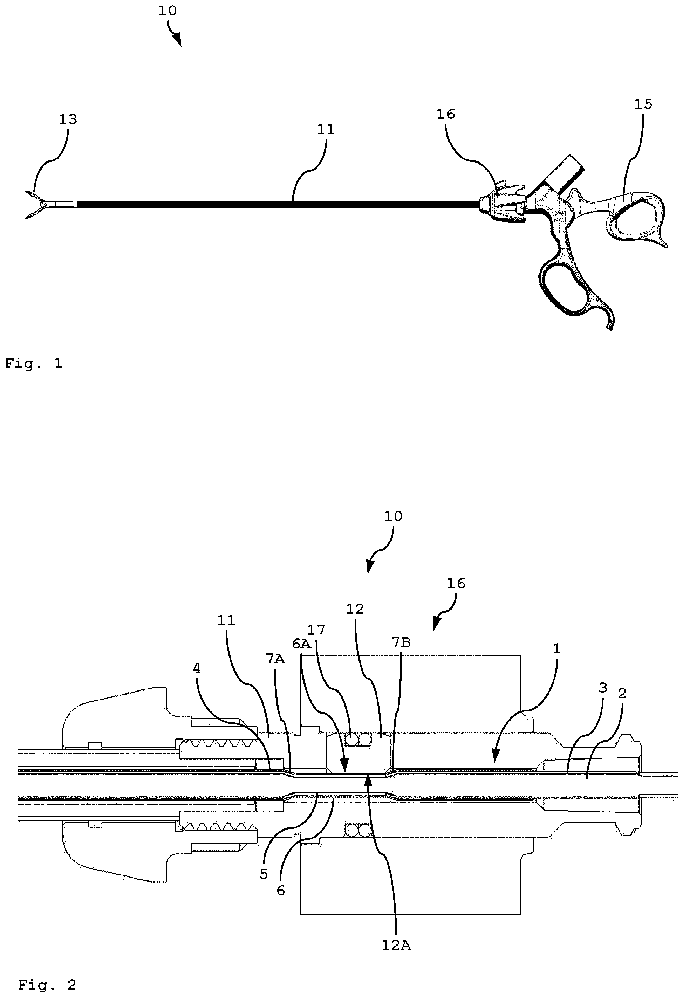

In Fig. 1 ist ein eines handgeführtes chirurgisches Instrument 10 schematisch dargestellt. Das chirurgische Instrument umfasst ein Kraftübertragungselement (hier nicht dargestellt, siehe Figs. 2 bis 10), einen Rohrschaft 11, ein Sicherungselement (hier nicht dargestellt, siehe Fig. 2), ein Werkzeug bzw. Zubehör 13, einen Handgriff 14, einen beweglichen Griffschenkel 15 und eine Zubehörschnittstelle 16.

Das Kraftübertragungselement ist in dem Rohrschaft 11 aufgenommen und gelagert (siehe Fig. 2). Das Sicherungselement sichert das Kraftübertragungselement gegen Verdrehen und zusätzlich oder alternativ gegen axiale Verschiebung gegenüber dem Rohrschaft 11 (siehe Fig. 2). Das Werkzeug 13 ist hier als Greifer ausgebildet, welcher über das Kraftübertragungselement mit bipolarem elektrischem Strom versorgt werden kann. Das chirurgische Instrument ist hier zur händischen Führung durch einen Nutzer (z. B. Chirurg) mit einem Handgriff 14 ausgestattet. Alternativ kann das chirurgische Instrument 10 zu Führung durch einen Roboter mit einer entsprechenden Anschlussschnittstelle für den Roboter ausgestattet sein (nicht dargestellt). Der bewegliche Griffschenkel 15 dient der manuellen Krafteinleitung. Dabei wird die auf den Griffschenkel 15 aufgebrachte Kraft durch den Griffschenkel 15 auf das Kraftübertragungselement übertragen. Das Kraftübertragungselement überträgt wiederum die axiale Kraft auf das Werkzeug / Zubehör. Zusätzlich werden über das Kraftübertragungselement zwei elektrische Pole eines bipolaren Generators (nicht dargestellt) mit dem Werkzeug verbunden. Über die Zubehörschnittstelle 16 kann das Werkzeug / Zubehör 13 mit dem Handgriff 14 lösbar mechanisch verbunden werden.

In Fig. 2 ist ein Längsschnitt durch das chirurgische Instrument 10 aus Fig. 1 im Bereich der Zubehörschnittstelle 16 schematisch dargestellt.

Das Kraftübertragungselement 1 ist in dem als Rundrohr ausgebildeten Rohrschaft 11 aufgenommen und geführt. Dabei wird das Kraftübertragungselement 1 durch das in dem Rohrschaft 11 geführte Sicherungselement 12 gesichert. Das Kraftübertragungselement 1 umfasst einen Stab 2, eine elektrische Isolierung 3 und eine Hülse 4.

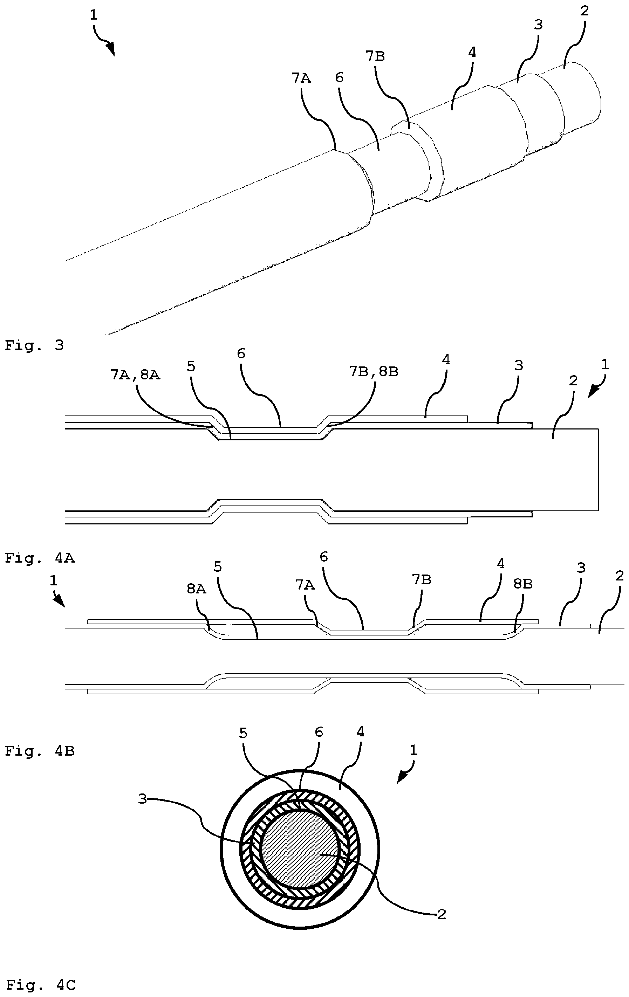

Der Stab 2 ist als Rundstab ausgeführt und aus Edelstahl gefertigt. Über den Stab 2 kann eine axiale Kraft, beispielsweise von dem beweglichen Griffschenkel (hier nicht dargestellt, siehe Fig. 1) an das Werkzeug (hier nicht dargestellt, siehe Fig. 1) übertragen werden. Ferner kann über den Stab 2 einer von zwei elektrischen Polen des bipolaren Generators mit dem Werkzeug verbunden werden. Der Stab 2 weist eine Vertiefung 5 auf. Die Vertiefung 5 kann unterschiedliche Formen aufweisen (siehe Figs. 3 bis 10).

Die elektrische Isolierung 3 ist aus PTFE gefertigt und umfänglich, hier zirkulär und konzentrisch, um den Stab angeordnet. Das Material könnte auch PFA oder ein ähnliches Material sein. Die elektrische Isolierung 3 ist mit dem Stab 2 mechanisch fest verbunden und trennt den Stab 2 elektrisch von der Hülse 4. Die elektrische Isolierung 3 ist auch im Bereich der Vertiefung 5 um den Stab 2 angeordnet und mit diesem fest mechanisch verbunden.

Die Hülse 4 ist aus Edelstahl gefertigt und umfänglich, hier zirkulär und konzentrisch, um die elektrische Isolierung 3 angeordnet. Die Hülse 4 weist im Bereich der Vertiefung 5 eine Prägung 6 auf, die in die Vertiefung 5 eingreift. Dabei steht die Hülse 4 über ihre gesamte Länge nur mit der elektrischen Isolierung 3 jedoch nicht mit dem Stab 2 in Kontakt. Über die Hülse 4 kann der andere der zwei elektrischen Pole des bipolaren Generators mit dem Werkzeug verbunden werden. Die Prägung 6 weist einen distalen Anschlag 7A und einen proximalen Anschlag 7B auf.

Das Sicherungselement 12 ist über eine Ringfeder 17 in distaler Richtung auf das Kraftübertragungselement 1 zu vorgespannt und greift in die Prägung 6 ein. Dabei liegt eine der Prägung zugewandte Fläche 12A des Sicherungselements 12 auf der äußeren Oberfläche der Prägung 6 an. Die der Prägung zugewandte Fläche 12A des Sicherungselements 12 kann im Wesentlichen eben sein und die äußere Oberfläche der Prägung 6 kann einen im Wesentlichen ebenen Abschnitt 6A umfassen, sodass die der Prägung zugewandte Fläche 12A des Sicherungselements 12 im Wesentlichen plan auf dem im Wesentlichen ebenen Abschnitt 6A der Prägung 6 anliegt. Dadurch ist der rotatorische Freiheitsgrad um die Längsachs der Hülse gesperrt und die Hülse 4 somit gegenüber dem Rohrschaft 11 vor Rotation / Verdrehen gesichert.

Eine distale Flanke des Sicherungselements 12 begrenzt den translatorischen Freiheitsgrad der Hülse in der axialen Richtung gemeinsam mit dem distalen Anschlag 7A der Prägung 6. Eine proximale Flanke des Sicherungselements 12 begrenzt den translatorischen Freiheitsgrad der Hülse in der axialen Richtung gemeinsam mit dem proximalen Anschlag 7B der Prägung 6. In Fig. 2 ist die proximale Flanke des Sicherungselements 12 an dem proximalen Anschlag 7B der Prägung 6 anliegend dargestellt. Die Hülse 4 kann daher nicht weiter distal gegenüber dem Rohrschaft 11 verschoben werden.

Inwiefern der Stab 2 gegenüber der Hülse 4 translatorisch oder rotatorisch beschränkt oder blockiert ist, hängt von der jeweiligen Ausführungsform des Kraftübertragungselements 1 ab. Nachfolgend werden vier unterschiedliche Ausführungsformen des Kraftübertragungselements 1 beschreiben.

In den Figs. 3, 4A, 4B und 4C ist eine erste Ausführungsform des Kraftübertragungselements 1 schematisch dargestellt.

Die Vertiefung 5 in dem Stab 2 ist zirkulär umlaufend und weist sowie einen distalen Anschlag der Prägung 5 und einen proximalen Anschlag der Prägung auf. Beispielsweise kann die Vertiefung 5 in dem Stab 2 durch Drehen geschaffen werden.

Die elektrische Isolierung 3 liegt zirkulär und konzentrisch an dem Stab 2 auch im Bereich der Vertiefung 5 an.

Die Prägung 6 in der Hülse 4 ist zirkulär umlaufend mit einem distalen Anschlag 7A der Prägung 6 und einem proximalen Anschlag 7B der Prägung 6 und kann beispielsweise durch Rollprägen mit einer oder mehreren drehenden Walzen gebildet werden.

In Fig. 4A ist ein Längsschnitt durch eine erste Variante der ersten Ausführungsform des Kraftübertragungselements 1 schematisch dargestellt. Die Hülse 4 weist eine Prägung 6 auf, die in die gesamte Vertiefung 5 eingreift, wobei die Hülse 4 an der elektrischen Isolierung 3 anliegt. Der distale Anschlag 7A der Hülse 4 liegt an einem distalen Anschlag 8A des Stabs 2 bzw. der Isolierung 3 in diesem Bereich an. Ebenso liegt der proximale Anschlag 7B der Hülse 4 an einem proximalen Anschlag 8B des Stabs 2 bzw. der Isolierung 3 in diesem Bereich an. Der Freiheitsgrad in axialer Richtung des Stabs 2 gegenüber der Hülse 4 ist somit blockiert. Je nach Gleiteigenschaften zwischen elektrischer Isolierung 3 und Hülse 4 kann der Stab 2 mit der Isolierung 3 gegenüber der Hülse 4 rotiert werden.

In Fig. 4B ist ein Längsschnitt durch eine zweite Variante der ersten Ausführungsform des Kraftübertragungselements 1 schematisch dargestellt. Die Hülse 4 weist eine Prägung 6 auf, die nur in einem Abschnitt in die Vertiefung 5 eingreift, wobei die Hülse 4 an der elektrischen Isolierung 3 anliegt. Der distale Anschlag 7A der Hülse 4 liegt nicht an dem distalen Anschlag 8A des Stabs 2 bzw. der Isolierung 3 in diesem Bereich an. Ebenso liegt der proximale Anschlag 7B der Hülse 4 nicht an dem proximalen Anschlag 8B des Stabs 2 bzw. der Isolierung 3 in diesem Bereich an. Der Freiheitsgrad in axialer Richtung des Stabs 2 gegenüber der Hülse 4 ist somit nur zwischen den Anschlägen begrenzt aber nicht vollständig blockiert. Je nach Gleiteigenschaften zwischen elektrischer Isolierung 3 und Hülse 4 kann der Stab 2 mit der Isolierung 3 gegenüber der Hülse 4 rotiert werden.

In Fig. 4C ist ein Querschnitt durch die erste Ausführungsform des Kraftübertragungselements 1 im Bereich der Prägung 6 schematisch dargestellt. Die Prägung 6 liegt an der elektrischen Isolierung 3 zirkulär und konzentrisch an.

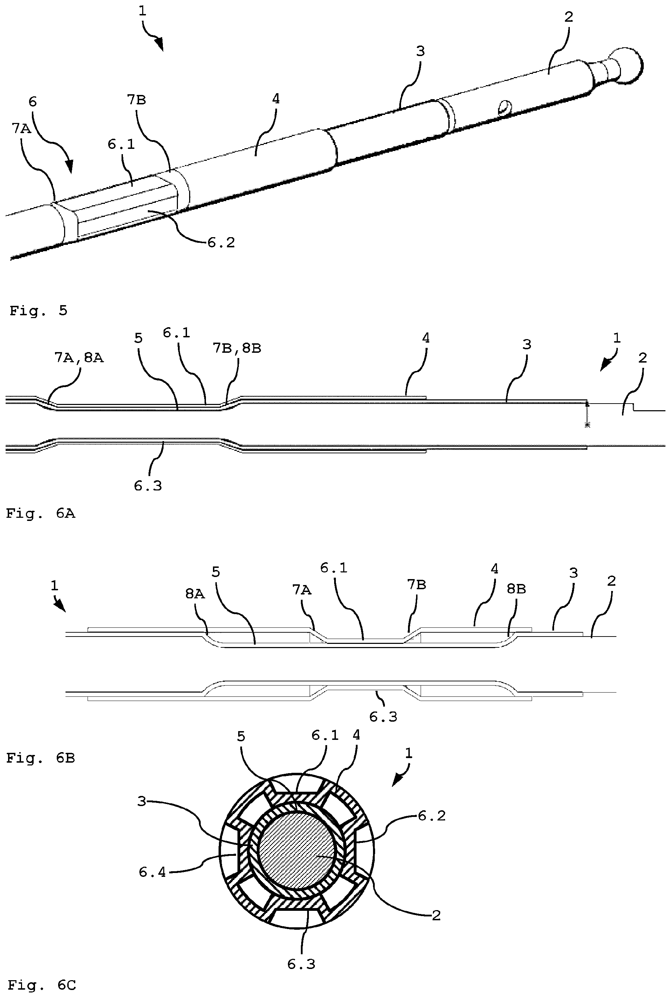

In den Figs. 5, 6A, 6B und 6C ist eine zweite Ausführungsform des Kraftübertragungselements 1 schematisch dargestellt.

Die Vertiefung 5 in dem Stab 2 ist zirkulär umlaufend und weist einen distalen Anschlag der Vertiefung 5 und einen proximalen Anschlag der Vertiefung 5 auf. Beispielsweise kann die Vertiefung 5 in dem Stab 2 durch Drehen geschaffen werden.

Die elektrische Isolierung 3 liegt zirkulär und konzentrisch an dem Stab 2 auch im Bereich der Vertiefung 5 an.

Die Prägung 6 in der Hülse 4 umfasst hier vier über den Umfang gleichmäßig verteilte ebene Prägungen 6.1, 6.2 (zwei der vier ebenen Prägungen nicht dargestellt) sowie einen distalen Anschlag 7A der Prägung 6 und einen proximalen Anschlag 7B der Prägung 6. Die ebenen Prägungen 6.1, 6.2 können beispielsweise durch Prägen mit einem oder mehreren Stempeln gebildet werden.

In Fig. 6A ist ein Längsschnitt durch eine erste Variante der zweiten Ausführungsform des Kraftübertragungselements 1 schematisch dargestellt. Die Hülse 4 weist die vier ebenen Prägungen 6.1, 6.3 (zwei der vier ebenen Prägungen nicht dargestellt) auf, die in die gesamte Vertiefung 5 eingreifen, wobei die Hülse 4 an der elektrischen Isolierung 3 anliegt. Der distale Anschlag 7A der Hülse 4 liegt an dem distalen Anschlag 8A des Stabs 2 bzw. der Isolierung 3 in diesem Bereich an. Ebenso liegt der proximale Anschlag 7B der Hülse 4 an dem proximalen Anschlag 8B des Stabs 2 bzw. der Isolierung 3 in diesem Bereich an. Der Freiheitsgrad in axialer Richtung des Stabs 2 gegenüber der Hülse 4 ist somit blockiert. Je nach Gleiteigenschaften zwischen elektrischer Isolierung 3 und Hülse 4 kann der Stab 2 mit der Isolierung 3 gegenüber der Hülse 4 rotiert werden. Wobei die Rotation auch blockiert werden kann.

In Fig. 6B ist ein Längsschnitt durch eine zweite Variante der zweiten Ausführungsform des Kraftübertragungselements 1 schematisch dargestellt. Die Hülse 4 weist die vier ebenen Prägungen 6.1, 6.3 (zwei der vier ebenen Prägungen nicht dargestellt) auf, die nur in einem Abschnitt in die Vertiefung 5 eingreifen, wobei die Hülse 4 an der elektrischen Isolierung 3 anliegt. Der distale Anschlag 7A der Hülse 4 liegt nicht an dem distalen Anschlag 8A des Stabs 2 bzw. der Isolierung 3 in diesem Bereich an. Ebenso liegt der proximale Anschlag 7B der Hülse 4 nicht an dem proximalen Anschlag 8B des Stabs 2 bzw. der Isolierung 3 in diesem Bereich an. Der Freiheitsgrad in axialer Richtung des Stabs 2 gegenüber der Hülse 4 ist somit nur zwischen den Anschlägen begrenzt aber nicht vollständig blockiert. Je nach Gleiteigenschaften zwischen elektrischer Isolierung 3 und Hülse 4 kann der Stab 2 mit der Isolierung 3 gegenüber der Hülse 4 rotiert werden. Wobei die Rotation auch blockiert werden kann.

In Fig. 6C ist ein Querschnitt durch die zweite Ausführungsform des Kraftübertragungselements 1 im Bereich der ebenen Prägungen 6.1, 6.2, 6.3, 6.4 schematisch dargestellt. Die ebenen Prägungen 6.1-6.4 liegen an der elektrischen Isolierung 3 an und können beispielsweise durch Prägen mit einem oder mehreren Stempeln gebildet werden.

Ein in eine der ebenen Prägungen 6.1, 6.2, 6.3, 6.4 eingreifendes Sicherungselement mit ebener der Prägung zugewandter Fläche blockiert den rotatorischen Freiheitsgrad der Hülse 4, jedoch nicht des Stabs 2 gegenüber dem Rohrschaft.

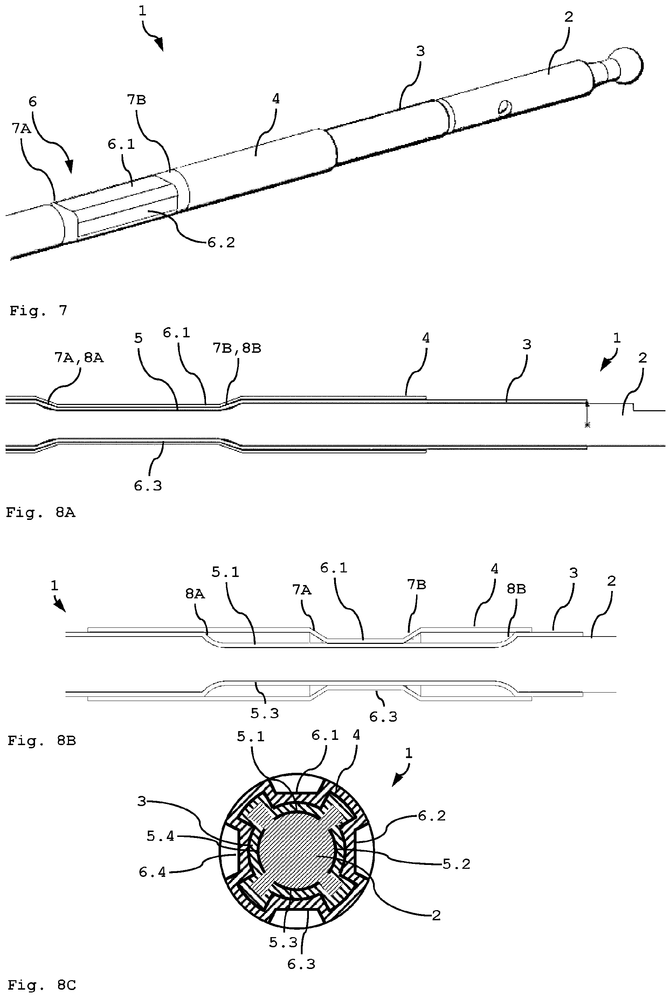

In den Figs. 7, 8A, 8B und 8C ist eine dritte Ausführungsform des Kraftübertragungselements 1 schematisch dargestellt.

Der Stab 2 umfasst hier vier über den Umfang gleichmäßig verteilte Vertiefungen, sowie einen distalen Anschlag der Vertiefung und einen proximalen Anschlag der Vertiefung.

Beispielsweise können die Vertiefungen in dem Stab 2 durch Fräsen geschaffen werden.

Die elektrische Isolierung 3 liegt zirkulär und konzentrisch an dem Stab 2 auch im Bereich der Vertiefungen an.

Die Prägung 6 in der Hülse 4 umfasst hier vier über den Umfang gleichmäßig verteilte ebene Prägungen 6.1, 6.2 (zwei der vier ebenen Prägungen nicht dargestellt), sowie einen distalen Anschlag 7A der Prägung 6 und einen proximalen Anschlag 7B der Prägung 6. Die ebenen Prägungen 6.1, 6.2 können beispielsweise durch Prägen mit einem oder mehreren Stempeln gebildet werden.

In Fig. 8A ist ein Längsschnitt durch eine erste Variante der dritten Ausführungsform des Kraftübertragungselements 1 schematisch dargestellt. Die Hülse 4 weist die vier ebenen Prägungen 6.1, 6.3 (zwei der vier ebenen Prägungen nicht dargestellt) auf, die jeweils in eine der vier Vertiefungen 5.1, 5.3 (zwei der vier Vertiefungen nicht dargestellt) gesamt eingreifen, wobei die Hülse 4 an der elektrischen Isolierung 3 anliegt. Der distale Anschlag 7A der Hülse 4 liegt an dem distalen Anschlag 8A des Stabs 2 bzw. der Isolierung 3 in diesem Bereich an. Ebenso liegt der proximale Anschlag 7B der Hülse 4 an dem proximalen Anschlag 8B des Stabs 2 bzw. der Isolierung 3 in diesem Bereich an. Der Freiheitsgrad in axialer Richtung des Stabs 2 gegenüber der Hülse 4 ist somit blockiert. Je nach Gleiteigenschaften zwischen elektrischer Isolierung 3 und Hülse 4 kann der Stab 2 mit der Isolierung 3 gegenüber der Hülse 4 rotiert werden.

In Fig. 8B ist ein Längsschnitt durch eine zweite Variante der dritten Ausführungsform des Kraftübertragungselements 1 schematisch dargestellt. Die Hülse 4 weist die vier ebenen Prägungen 6.1, 6.3 (zwei der vier ebenen Prägungen nicht dargestellt) auf, die jeweils nur in einem Abschnitt in die entsprechende Vertiefung 5.1, 5.3 (zwei der vier Vertiefungen nicht dargestellt) eingreifen, wobei die Hülse 4 an der elektrischen Isolierung 3 anliegt. Der distale Anschlag 7A der Hülse 4 liegt nicht an dem distalen Anschlag 8A des Stabs 2 bzw. der Isolierung 3 in diesem Bereich an. Ebenso liegt der proximale Anschlag 7B der Hülse 4 nicht an dem proximalen Anschlag 8B des Stabs 2 bzw. der Isolierung 3 in diesem Bereich an. Der Freiheitsgrad in axialer Richtung des Stabs 2 gegenüber der Hülse 4 ist somit nur zwischen den Anschlägen begrenzt aber nicht vollständig blockiert. Je nach Gleiteigenschaften zwischen elektrischer Isolierung 3 und Hülse 4 kann der Stab 2 mit der Isolierung 3 gegenüber der Hülse 4 rotiert werden.

In Fig. 8C ist ein Querschnitt durch die zweite Ausführungsform des Kraftübertragungselements 1 im Bereich der ebenen Prägungen 6.1, 6.2, 6.3, 6.4 schematisch dargestellt. Die ebenen Prägungen 6.1-6.4 greifen in die jeweiligen Vertiefungen 5.1-5.4 ein und liegen an der elektrischen Isolierung 3 an. Die ebenen Prägungen 6.1-6.4 können beispielsweise durch Prägen mit einem oder mehreren Stempeln gebildet werden. Die Vertiefungen können beispielsweise durch Fräsen gebildet werden.

Ein in eine der ebenen Prägungen 6.1, 6.2, 6.3, 6.4 eingreifendes Sicherungselement mit ebener der Prägung zugewandter Fläche blockiert den rotatorischen Freiheitsgrad der Hülse 4, jedoch nicht des Stabs 2 gegenüber dem Rohrschaft.

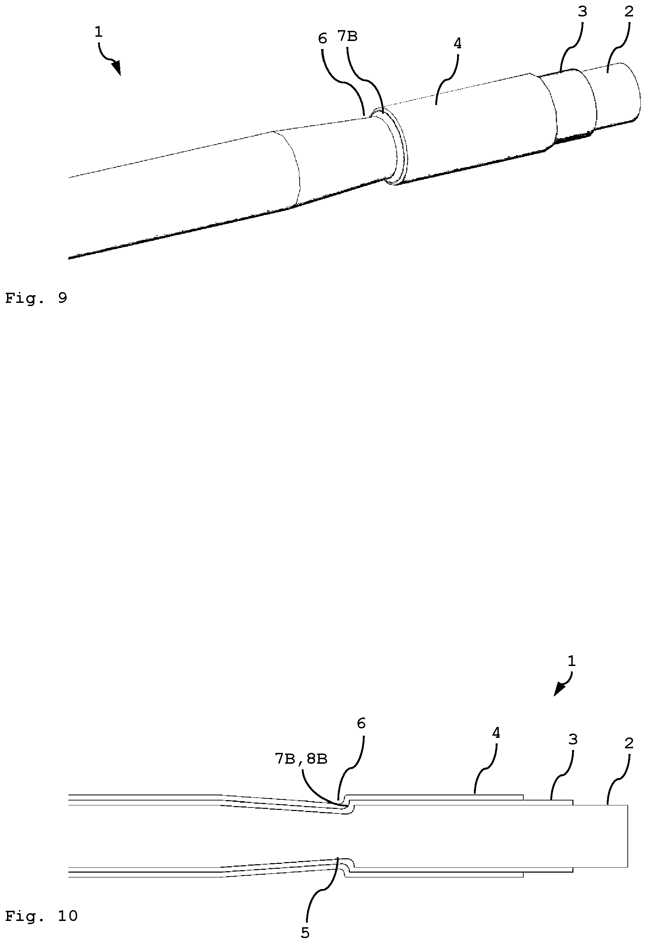

In den Figs. 9 und 10 ist eine vierte Ausführungsform des Kraftübertragungselements 1 schematisch dargestellt.

Die Vertiefung in dem Stab 2 ist zirkulär umlaufend und weist nur einen proximalen Anschlag der Vertiefung 5 auf. Dabei läuft die Vertiefung unter einem vorbestimmten Winkel gegenüber der axialen Richtung in distaler Richtung aus. Beispielsweise kann die Vertiefung 5 in dem Stab 2 durch Drehen geschaffen werden.

Die elektrische Isolierung 3 liegt zirkulär und konzentrisch an dem Stab 2 auch im Bereich der Vertiefung 5 an.

Die Prägung 6 in der Hülse 4 ist zirkulär umlaufend mit nur einem proximalen Anschlag 7B und kann beispielsweise durch Rollprägen mit einer oder mehreren drehenden Walzen gebildet werden, die gegenüber der axialen Richtung um einen entsprechenden Winkel gekippt sind.

In Fig. 10 ist ein Längsschnitt durch die vierte Ausführungsform des Kraftübertragungselements 1 schematisch dargestellt. Die Hülse 4 weist eine Prägung 6 auf, die in die gesamte Vertiefung 5 eingreift, wobei die Hülse 4 an der elektrischen Isolierung 3 anliegt. Der proximale Anschlag 7B der Hülse 4 liegt an dem proximalen Anschlag 8B des Stabs 2 bzw. der Isolierung 3 in diesem Bereich an. Der Freiheitsgrad in axialer Richtung des Stabs 2 gegenüber der Hülse 4 ist somit zumindest in distaler Richtung begrenzt. Je nach Gleiteigenschaften zwischen elektrischer Isolierung 3 und Hülse 4 kann der Stab 2 mit der Isolierung 3 gegenüber der Hülse 4 rotiert werden.

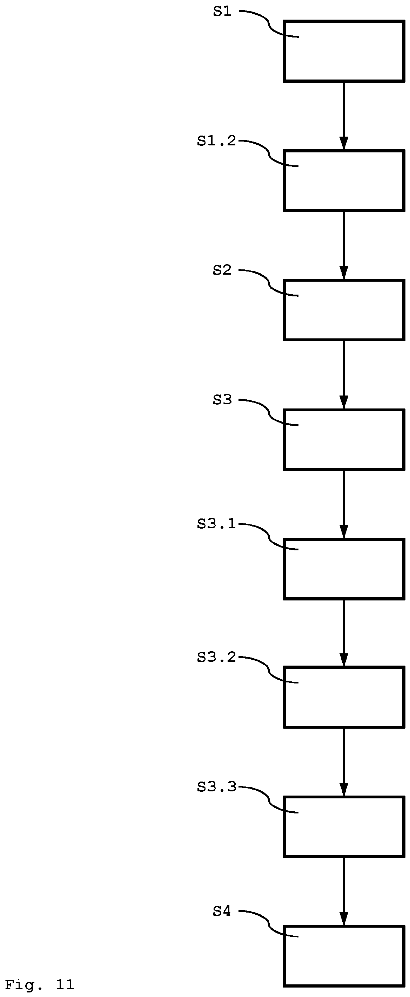

In Fig. 11 ist eine Ausführungsform des Verfahrens zur Herstellung des Kraftübertragungselements für chirurgische Instrumente, insbesondere eines Kraftübertragungselements 1 aus den Fig. 2 bis 10, schematisch dargestellt. Das Verfahren umfasst die Schritte Bereitstellen S1, Aufbringen S2, umfängliches Anordnen S3 und Einprägen S3.

Im Schritt des Bereitstellens S1 wird ein elektrisch leitfähiger zur Kraftübertragung ausgebildeter Stab bereitgestellt, der eine Vertiefung aufweist. Der Schritt des Bereitstellens S1 umfasst den Unterschritt Schaffen S1.2.

Im Schritt des Schaffens S1.2 wird die Vertiefung in dem Stab geschaffen. Dabei wird die Vertiefung insbesondere durch Umformen, beispielsweise Pressen, oder durch Zerspanen, beispielsweise Fräsen oder Drehen, geschaffen. Es kann nur ein (proximaler oder distaler) Anschlag der Vertiefung, optional im (distal oder proximal) auslaufender Vertiefung, oder es können zwei Anschläge (ein proximaler und ein distaler Anschlag) der Vertiefung geschaffen werden.

Im Schritt des Aufbringens S2 wird einer elektrischen Isolierung umfänglich, bevorzugt zirkulär und besonders bevorzugt konzentrisch, über eine äußere Oberfläche und zumindest abschnittsweise in axialer Richtung entlang des Stabs derart aufgebracht, dass die Isolierung sich zumindest entlang der Vertiefung erstreckt. Insbesondere umfasst der Schritt des Aufbringens S2 ein Aufschrumpfen eines Schrumpfschlauchs (z. B. aus PTFE) auf den Stab oder ein Beschichten des Stabs (z. B. mit einem polymeren oder keramischen Werkstoff) umfasst.

Im Schritt des umfänglichen Anordnens S3 wird eine elektrisch leitfähige Hülse um die Isolierung zumindest abschnittsweise in axialer Richtung entlang der elektrischen Isolierung umfänglich, bevorzugt zirkulär und besonders bevorzugt konzentrisch, angeordnet. Der Schritt des umfänglichen Anordnens S3 umfasst die Unterschritte Erwärmen S3.1, Aufschieben S3.2 und Abkühlen S3.3.

Im Schritt des Erwärmens S3.1 wird die Hülse erwärmt bis durch thermische Dehnung ein Innendurchmesser der Hülse größer als ein Außendurchmesser der elektrischen Isolierung ist.

Im Schritt des Aufschiebens S3.2 wird die erwärmte Hülse auf die elektrische Isolierung aufgeschoben. Dabei wird die Hülse soweit (in axialer Richtung) aufgeschoben, bis sie zumindest in einem Bereich über der Vertiefung angeordnet ist.

Im Schritt des Abkühlens S3.3 wird die erwärmte und aufgeschobene Hülse abgekühlt. Dies kann beispielsweise durch Wärmestrahlung oder Konvektion gegenüber ruhiger oder bewegter Umgebungsluft oder durch Abschrecken mit einem Kühlmedium erfolgen.

Im Schritt des Einprägens S4 wird eine zumindest abschnittsweise in die Vertiefung eingreifende und zum Eingriff mit einem Sicherungselement eines chirurgischen Instruments ausgebildete Prägung in die Hülse eingeprägt. Das Einprägen der Prägung in die Hülse kann mittels Rollprägen mit einer oder mehreren Prägewalzen parallel zur axialen Richtung oder verkippt gegenüber dieser oder mittels Prägen mit einem oder mehreren in radialer Richtung auf die Hülse einwirkenden Stempeln erfolgen.

### BEZUGSZEICHENLISTE

- 1: Kraftübertragungselement
- 2: Stab
- 3: elektrische Isolierung
- 4: Hülse
- 5: Vertiefung
- 5.1-5.4: einzelne Vertiefungen
- 6: Prägung
- 6A: im Wesentlichen ebener Abschnitt
- 6.1-6.4: ebene Prägung
- 7A-7B: Anschlag der Prägung
- 8A-8B: Anschlag der Vertiefung
- 10: chirurgisches Instrument
- 11: Rohrschaft
- 12: Sicherungselement
- 12A: der Prägung zugewandte Fläche
- 13: Werkzeug / Zubehör
- 14: Handgriff
- 15: beweglicher Griffschenkel
- 16: Zubehörschnittstelle
- 17: Ringfeder

## Patentansprüche

1. Kraftübertragungselement (1) für chirurgische Instrumente, mit:
einem elektrisch leitfähigen zur Kraftübertragung ausgebildeten Stab (2);
einer elektrischen Isolierung (3), die sich umfänglich über eine äußere Oberfläche und zumindest abschnittsweise in axialer Richtung entlang des Stabs (2) erstreckt; und
einer elektrisch leitfähigen Hülse (4), die umfänglich um die elektrische Isolierung (3) angeordnet ist und sich zumindest abschnittsweise in axialer Richtung entlang der elektrischen Isolierung (3) erstreckt,
wobei der Stab zumindest eine Vertiefung (5) aufweist und die Isolierung (3) sich zumindest entlang der Vertiefung (5) erstreckt, und
wobei die Hülse (4) eine Prägung (6) aufweist, die sich zumindest abschnittsweise in die Vertiefung (5) erstreckt und zum Eingriff mit einem Sicherungselement (12) eines chirurgischen Instruments (10) ausgebildet ist.

2. Kraftübertragungselement (1) nach Anspruch 1,
**dadurch gekennzeichnet,**
**dass** der Stab (2) als Rundstab ausgebildet ist, wobei sich die elektrische Isolierung (3) zumindest abschnittsweise zirkulär entlang der Oberfläche des Stabs (2) erstreckt, und wobei die Hülse (4) als Rundrohr ausgebildet ist und sich zumindest abschnittsweise zirkulär entlang der Oberfläche der elektrischen Isolierung (3) erstreckt.

3. Kraftübertragungselement (1) nach einem der vorstehenden Ansprüche,
**dadurch gekennzeichnet,**
**dass** die Prägung (6) zum Sperren wenigstens des rotatorischen Freiheitsgrads um eine Achse parallel zur axialen Richtung der Hülse (4) bei Eingriff eines Sicherungselements (12) ausgebildet ist.

4. Kraftübertragungselement (1) nach Anspruch 3,
**dadurch gekennzeichnet,**
**dass** die Prägung (6) wenigstens einen im Wesentlichen ebenen Abschnitt (6A) zur im Wesentlichen plan anliegenden Kontaktierung einer ebenen Fläche (12A) eines Sicherungselements (12) aufweist, welcher parallel zu der axialen Richtung der Hülse (4) verläuft und gegenüber der äußeren Oberfläche der Hülse (4) radial weiter innen liegt.

5. Kraftübertragungselement (1) nach Anspruch 4,
**dadurch gekennzeichnet,**
**dass** die Vertiefung (5) wenigstens einen im Wesentlichen ebenen Abschnitt (5A) parallel zu der axialen Richtung des Stabs (2) aufweist, welcher gegenüber der äußeren Oberfläche des Stabs (2) in radialer Richtung weiter innen liegt und mit dem im Wesentlichen ebenen Abschnitt (6A) der Prägung (6) aneinander anliegend, insbesondere plan anliegend, kontaktiert ist.

6. Kraftübertragungselement (1) nach einem der vorstehenden Ansprüche,
**dadurch gekennzeichnet,**
**dass** die Prägung (6) zum Begrenzen oder Sperren wenigstens des translatorischen Freiheitsgrads in einer Richtung parallel zu der Hülse (4) bei Eingriff eines Sicherungselements (12) ausgebildet ist, wobei die Prägung (6) insbesondere wenigstens einen Anschlag (7A, 7B) zum Sperren oder Begrenzen wenigstens des translatorischen Freiheitsgrads aufweist.

7. Kraftübertragungselement (1) nach einem der vorstehenden Ansprüche,
**dadurch gekennzeichnet,**
**dass** die Vertiefung (5) wenigstens einen Anschlag (8A, 8B) aufweist, der zum Begrenzen oder Sperren wenigstens des translatorischen Freiheitsgrads in einer Richtung parallel zu dem Stab (2) gemeinsam mit der Prägung (6) ausgebildet ist.

8. Kraftübertragungselement (1) nach einem der vorstehenden Ansprüche,
**dadurch gekennzeichnet,**
**dass** die elektrische Isolierung (3) zumindest im Bereich der Prägung (6) Gleiteigenschaften auf ihrer äußeren Oberfläche und/oder die Hülse (4) zumindest im Bereich der Prägung (6) Gleiteigenschaften auf ihrer inneren Oberfläche aufweist.

9. Chirurgisches Instrument (10), mit:
einem Kraftübertragungselement (1) gemäß einem der vorstehenden Ansprüche;
einem Rohrschaft (11), in welchem das Kraftübertragungselement (1) aufgenommen ist;
einem Sicherungselement (12), das in dem Rohrschaft (11) gelagert ist und in die Prägung (6) der Hülse (4) des Kraftübertragungselements (1) eingreift;
einem Werkzeug (13), das mittels des Kraftübertragungselements (1) bewegbar ist; und
einer Betätigungsschnittstelle, die zum Betätigen des Kraftübertragungselements ausgebildet ist.

10. Chirurgisches Instrument (10) nach Anspruch 9,
**dadurch gekennzeichnet,**
**dass** der Rohrschaft (11) als Rundrohr ausgebildet ist, wobei der Stab (2) des Kraftübertragungselements (1) als Rundstab ausgebildet ist und sich die elektrische Isolierung (3) zumindest abschnittsweise zirkulär entlang der Oberfläche des Stabs (2) erstreckt, wobei die Hülse (4) als Rundrohr ausgebildet ist und sich zumindest abschnittsweise zirkulär entlang der Oberfläche der elektrischen Isolierung (3) erstreckt, und wobei das Kraftübertragungselement (1) konzentrisch in dem Rohrschaft (11) angeordnet ist.

11. Chirurgisches Instrument (10) nach Anspruch 9 oder 10,
**dadurch gekennzeichnet,**
**dass** das Sicherungselement (11) auf seiner der Prägung (6) zugewandten Oberfläche und/oder die Prägung (6) auf ihrer äußeren Oberfläche Gleiteigenschaften aufweist.

12. Verfahren zur Herstellung eines Kraftübertragungselements (1) für chirurgische Instrumente, insbesondere eines Kraftübertragungselements (1) gemäß einem der Ansprüche 1 bis 7, umfassend die folgenden Schritte:
Bereitstellen (S1) eines elektrisch leitfähigen zur Kraftübertragung ausgebildeten Stabs (2), der eine Vertiefung (5) aufweist;
Aufbringen (S2) einer elektrischen Isolierung (3) umfänglich über eine äußere Oberfläche und zumindest abschnittsweise in axialer Richtung entlang des Stabs (2), sodass die Isolierung (3) sich zumindest entlang der Vertiefung (5) erstreckt;
umfängliches Anordnen (S3) einer elektrisch leitfähigen Hülse (4) um die Isolierung (3) zumindest abschnittsweise in axialer Richtung entlang der elektrischen Isolierung (3); und
Einprägen (S4) einer zumindest abschnittsweise in die Vertiefung (5) eingreifenden und zum Eingriff mit einem Sicherungselement (12) eines chirurgischen Instruments (10) ausgebildeten Prägung (6) in die Hülse (4).

13. Verfahren nach Anspruch 12,
**dadurch gekennzeichnet,**
**dass** das Bereitstellen (S1) einen Schritt des Schaffens (S1.2) der Vertiefung (5) in dem Stab (2) umfasst, insbesondere durch Umformen oder Zerspanen.

14. Verfahren nach Anspruch 12 oder 13,
**dadurch gekennzeichnet,**
**dass** das Aufbringen (S2) der Isolierung (3) ein Aufschrumpfen eines Schrumpfschlauchs auf den Stab (2) oder ein Beschichten des Stabs (2) umfasst.

15. Verfahren nach einem der Ansprüche 12 bis 14,
**dadurch gekennzeichnet,**
**dass** der Schritt des umfänglichen Anordnens (S3) die folgenden Schritte umfasst:
Erwärmen (S3.1) der Hülse (4) bis durch thermische Dehnung ein Innendurchmesser der Hülse (4) größer als ein Außendurchmesser der elektrischen Isolierung (3) ist;
Aufschieben (S3.2) der erwärmten Hülse (4) auf die elektrische Isolierung (3); und
Abkühlen (S3.3) der erwärmten und aufgeschobenen Hülse (4).

## Claims

1. A force transmission element (1) for surgical instruments, with:
an electrically conductive rod (2) designed for force transmission;
an electrical insulation (3), which extends circumferentially over an outer surface and at least in sections in the axial direction along the rod (2); and
an electrically conductive sleeve (4), which is arranged circumferentially around the electrical insulation (3) and extends at least in sections in the axial direction along the electrical insulation (3),
wherein the rod has at least one recess (5) and the insulation (3) extends at least along the recess (5), and
wherein the sleeve (4) has an embossment (6), which extends at least in sections into the recess (5) and which is designed to engage with a securing element (12) of a surgical instrument (10).

2. The force transmission element (1) according to claim 1,
**characterised in**
**that** the rod (2) is designed as a round rod, wherein the electrical insulation (3) extends at least in sections circularly along the surface of the rod (2), and wherein the sleeve (4) is designed as a round tube and extends at least in sections circularly along the surface of the electrical insulation (3).

3. The force transmission element (1) according to one of the preceding claims,
**characterised in**
**that** the embossment (6) is designed to block at least the rotational degree of freedom about an axis parallel to the axial direction of the sleeve (4) upon engagement of a securing element (12).

4. The force transmission element (1) according to claim 3,
**characterised in**
**that** the embossment (6) has at least one substantially flat section (6A) for making substantially planar-lying contact with a flat surface (12A) of a securing element (12), which runs parallel to the axial direction of the sleeve (4) and lies radially further inwards with respect to the outer surface of the sleeve (4).

5. The force transmission element (1) according to claim 4,
**characterised in**
**that** the recess (5) has at least one substantially flat section (5A) parallel to the axial direction of the rod (2), which lies further inwards in the radial direction with respect to the outer surface of the rod (2) and is contacted with the substantially flat section (6A) of the embossment (6) so as to lie, in particular lie planar, on one another.

6. The force transmission element (1) according to one of the preceding claims,
**characterised in**
**that** the embossment (6) is designed for limiting or blocking at least the translational degree of freedom in a direction parallel to the sleeve (4) upon engagement of a securing element (12), wherein the embossment (6) has in particular at least one stop (7A, 7B) for blocking or limiting at least the translational degree of freedom.

7. The force transmission element (1) according to one of the preceding claims,
**characterised in**
**that** the recess (5) has at least one stop (8A, 8B) which is designed to limit or block at least the translational degree of freedom in a direction parallel to the rod (2) together with the embossment (6).

8. The force transmission element (1) according to one of the preceding claims,
**characterised in**
**that** the electrical insulation (3) has sliding properties on its outer surface at least in the region of the embossment (6) and/or the sleeve (4) has sliding properties on its inner surface at least in the region of the embossment (6).

9. A surgical instrument (10), with:
a force transmission element (1) according to one of the preceding claims;
a tubular shaft (11) in which the force transmission element (1) is received;
a securing element (12), which is supported in the tubular shaft (11) and which engages into the embossment (6) of the sleeve (4) of the force transmission element (1);
a tool (13) which is movable by means of the force transmission element (1); and
an actuating interface which is designed to actuate the force transmission element.

10. The surgical instrument (10) according to claim 9,
**characterised in**
**that** the tubular shaft (11) is designed as a round tube, wherein the rod (2) of the force transmission element (1) is designed as a round rod and the electrical insulation (3) extends at least in sections circularly along the surface of the rod (2), wherein the sleeve (4) is designed as a round tube and extends at least in sections circularly along the surface of the electrical insulation (3), and wherein the force transmission element (1) is arranged concentrically in the tubular shaft (11).

11. The surgical instrument (10) according to claim 9 or 10,
**characterised in**
**that** the securing element (11) has sliding properties on its surface facing the embossment (6) and/or the embossment (6) has sliding properties on its outer surface.

12. A method for manufacturing a force transmission element (1) for surgical instruments, in particular a force transmission element (1) according to one of claims 1 to 7, comprising the following steps:
providing (S1) an electrically conductive rod (2) which is designed for force transmission and which has a recess (5);
applying (S2) an electrical insulation (3) circumferentially over an outer surface and at least in sections in the axial direction along the rod (2) such that the insulation (3) extends at least along the recess (5);
circumferentially arranging (S3) an electrically conductive sleeve (4) around the insulation (3) at least in sections in the axial direction along the electrical insulation (3); and
impressing (S4) into the sleeve (4) an embossment (6) which engages at least in sections into the recess (5) and which is designed to engage with a securing element (12) of a surgical instrument (10).

13. The method according to claim 12,
**characterised in**
**that** providing (S1) comprises a step of creating (S1.2) the recess (5) in the rod (2), in particular by reshaping or machining.

14. The method according to claim 12 or 13,
**characterised in**
**that** applying (S2) the insulation (3) comprises shrinking a shrink tube onto the rod (2) or coating the rod (2).

15. The method according to one of claims 12 to 14,
**characterised in**
**that** the step of circumferentially arranging (S3) comprises the following steps:
heating (S3.1) the sleeve (4) until, due to thermal expansion, an inner diameter of the sleeve (4) is larger than an outer diameter of the electrical insulation (3);
sliding (S3.2) the heated sleeve (4) onto the electrical insulation (3); and
cooling (S3.3) the heated sleeve (4) that has been pushed on.

## Revendications

1. Élément de transmission de force (1) pour instruments chirurgicaux, ayant :
une tige conductrice d'électricité (2) conçue pour la transmission de force ;
une isolation électrique (3), qui s'étend circonférentiellement sur une surface extérieure et au moins en sections dans la direction axiale le long de la tige (2) ; et
une enveloppe conductrice d'électricité (4), qui est disposée circonférentiellement autour de l'isolation électrique (3) et s'étend au moins en sections dans la direction axiale le long de l'isolation électrique (3),
dans lequel la tige présente au moins un évidement (5) et l'isolant (3) s'étend au moins le long de l'évidement (5), et
dans lequel l'enveloppe (4) présente un bossage (6), qui s'étend au moins en sections dans l'évidement (5) et qui est conçue pour venir en prise avec un élément de fixation (12) d'un instrument chirurgical (10).

2. Élément de transmission de force (1) selon la revendication 1,
**caractérisé en ce**
**que** la tige (2) est conçue comme une tige ronde, dans lequel l'isolation électrique (3) s'étend au moins en sections de manière circulaire le long de la surface de la tige (2), et dans lequel l'enveloppe (4) est conçue comme un tube rond et s'étend au moins en sections de manière circulaire le long de la surface de l'isolation électrique (3).

3. Élément de transmission de force (1) selon l'une des revendications précédentes,
**caractérisé en ce**
**que** le bossage (6) est conçu pour bloquer au moins le degré de liberté de rotation autour d'un axe parallèle à la direction axiale de l'enveloppe (4) lors de la mise en prise d'un élément de fixation (12).

4. Élément de transmission de force (1) selon la revendication 3,
**caractérisé en ce**
**que** le bossage (6) présente au moins une section sensiblement plate (6A) destinée à entrer en contact de façon sensiblement plane avec une surface plate (12A) d'un élément de fixation (12), qui est parallèle à la direction axiale de l'enveloppe (4) et se trouve radialement plus à l'intérieur par rapport à la surface extérieure de l'enveloppe (4).

5. Élément de transmission de force (1) selon la revendication 4,
caractérisé en ce
l'évidement (5) présente au moins une section sensiblement plate (5A) parallèle à la direction axiale de la tige (2), qui se trouve plus à l'intérieur dans la direction radiale par rapport à la surface extérieure de la tige (2) et qui est en contact avec la section sensiblement plate (6A) du bossage (6) de manière à se trouver, en particulier se trouver planaires, l'une sur l'autre.

6. Élément de transmission de force (1) selon l'une des revendications précédentes,
**caractérisé en ce**
**que** le bossage (6) est conçu pour limiter ou bloquer au moins le degré de liberté de translation dans une direction parallèle a l'enveloppe (4) lors de la mise en prise d'un élément de fixation (12), dans lequel le bossage (6) présente en particulier au moins une butée (7A, 7B) pour bloquer ou limiter au moins le degré de liberté de translation.

7. Élément de transmission de force (1) selon l'une des revendications précédentes,
**caractérisé en ce**
**que** l'évidement (5) présente au moins une butée (8A, 8B) qui est conçue pour limiter ou bloquer au moins le degré de liberté de translation dans une direction parallèle à la tige (2) avec le bossage (6).

8. Élément de transmission de force (1) selon l'une des revendications précédentes,
**caractérisé en ce**
**que** l'isolation électrique (3) présente des propriétés de glissement sur sa surface extérieure au moins dans la zone du bossage (6) et/ou que l'enveloppe (4) présente des propriétés de glissement sur sa surface intérieure au moins dans la zone du bossage (6).

9. Instrument chirurgical (10), ayant :
un élément de transmission de force (1) selon l'une des revendications précédentes ;
un arbre tubulaire (11) dans lequel l'élément de transmission de force (1) est reçu ;
un élément de fixation (12), qui est soutenu par l'arbre tubulaire (11) et qui vient en prise dans le bossage (6) de l'enveloppe (4) de l'élément de transmission de force (1) ;
un outil (13) qui est déplaçable au moyen de l'élément de transmission de force (1) ; et
une interface d'actionnement qui est conçue pour actionner l'élément de transmission de force.

10. Instrument chirurgical (10) selon la revendication 9,
**caractérisé en ce**
**que** l'arbre tubulaire (11) est conçu comme un tube rond, dans lequel la tige (2) de l'élément de transmission de force (1) est conçue comme une tige ronde et l'isolation électrique (3) s'étend au moins en sections de manière circulaire le long de la surface de la tige (2), dans lequel l'enveloppe (4) est conçue comme un tube rond et s'étend au moins en sections de manière circulaire le long de la surface de l'isolation électrique (3), et dans lequel l'élément de transmission de force (1) est disposé concentriquement dans l'arbre tubulaire (11).

11. Instrument chirurgical (10) selon la revendication 9 ou 10,
**caractérisé en ce**
**que** l'élément de fixation (11) présente des propriétés de glissement sur sa surface faisant face au bossage (6) et/ou que le bossage (6) présente des propriétés de glissement sur sa surface extérieure.

12. Procédé de fabrication d'un élément de transmission de force (1) pour instruments chirurgicaux, en particulier un élément de transmission de force (1) selon l'une des revendications 1 à 7, comprenant les étapes suivantes :
fournir (S1) une tige conductrice d'électricité (2) conçue pour la transmission d'une force et présentant un évidement (5) ;
appliquer (S2) une isolation électrique (3) de manière circonférentielle sur une surface extérieure et au moins en sections dans la direction axiale le long de la tige (2) de sorte que l'isolation (3) s'étend au moins le long de l'évidement (5) ;
disposer (S3) une enveloppe conductrice d'électricité (4) circonférentiellement autour de l'isolation (3) au moins en sections dans la direction axiale le long de l'isolation électrique (3) ; et
imprimer (S4) un bossage (6) dans l'enveloppe (4) qui vient en prise au moins en sections dans l'évidement (5) et qui est conçu pour venir en prise dans un élément de fixation (12) d'un instrument chirurgical (10).

13. Procédé selon la revendication 12,
**caractérisé en ce**
**que** fournir (S1) comprend une étape de création (S1.2) de l'évidement (5) dans la tige (2), notamment par remodelage ou usinage.

14. Procédé selon la revendication 12 ou 13,
**caractérisé en ce**
**qu'**appliquer (S2) l'isolant (3) comprend la rétraction d'une gaine thermorétractable sur la tige (2) ou le revêtement de la tige (2).

15. Procédé selon l'une des revendications 12 à 14,
caractérisé en ce
l'étape de disposer (S3) circonférentiellement comprend les étapes suivantes :
chauffer (S3.1) l'enveloppe (4) jusqu'à ce que, en raison de la dilatation thermique, un diamètre intérieur de l'enveloppe (4) est plus grand que le diamètre extérieur de l'isolation électrique (3) ;
faire glisser (S3.2) l'enveloppe (4) chauffée sur l'isolation électrique (3) ; et
refroidir (S3.3) l'enveloppe (4) chauffée qui a été enfoncée.
